# EUROPÄISCHE PATENTSCHRIFT

(11) **EP 3 068 331 B1**
(45) Veröffentlichungstag und Bekanntmachung des Hinweises auf die Patenterteilung: **18.08.2021**
(21) Anmeldenummer: 14795631.2
(22) Anmeldetag: 07.11.2014
(51) Int. Cl.: A61B 90/00

(54) **CHIRURGISCHE REFERENZIERUNGSVORRICHTUNG UND CHIRURGISCHES NAVIGATIONSSYSTEM**
SURGICAL REFERENCING DEVICE AND SURGICAL NAVIGATION SYSTEM
DISPOSITIF DE RÉFÉRENCEMENT CHIRURGICAL ET SYSTÈME DE NAVIGATION CHIRURGICAL

(30) Priorität: 11.11.2013 DE 102013112375
(43) Veröffentlichungstag der Anmeldung: 21.09.2016
(73) Patentinhaber: Aesculap AG, 78532 Tuttlingen (DE)
(72) Erfinder: PFEIFER, Tobias, 78713 Waldmössingen (DE); BROERS, Holger, 26670 Uplengen-Spols (DE); BENEKE, Sascha, 26835 Holtland-Nücke (DE)
(74) Vertreter: Hoeger, Stellrecht & Partner Patentanwälte mbB
(86) Internationale Anmeldenummer: PCT/EP2014/074019
(87) Internationale Veröffentlichungsnummer: WO 2015/067743

(56) Entgegenhaltungen:
- EP-A1- 1 518 508
- EP-A1- 2 179 692
- EP-A1- 2 198 801
- EP-A1- 2 298 215
- WO-A1-2011/023232
- WO-A1-2011/113482
- WO-A1-2012/068679
- US-A1- 2004 106 861

## Beschreibung

Die Erfindung betrifft eine chirurgische Referenzierungsvorrichtung, die von einem chirurgischen Navigationssystem verfolgt werden kann, umfassend eine Markiereinrichtung mit mindestens zwei oder mehr chirurgischen Markerelementen, die zum Reflektieren von Strahlung ausgebildet sind, wobei die Markerelemente eine Markerelementanordnung ausbilden, die eine Referenz am Körper eines Patienten definiert, sowie umfassend eine Befestigungseinrichtung zum Festlegen der Markiereinrichtung am Körper des Patienten. Außerdem betrifft die Erfindung ein chirurgisches Navigationssystem zum Verfolgen einer chirurgischen Referenzierungsvorrichtung im Raum, wobei das Navigationssystem eine Referenzierungsvorrichtung mit einer Markiereinrichtung aufweisend mindestens zwei oder mehr Markerelemente umfasst, eine Nachweisvorrichtung zum Detektieren von den Markerelementen reflektierter Strahlung und Bereitstellen diesbezüglicher Positionsdaten sowie eine Datenverarbeitungsvorrichtung zur Verarbeitung der Positionsdaten und darauf beruhender Ermittlung der Lage und der Orientierung der Referenzierungsvorrichtung im Raum.

Außerdem betrifft die Erfindung ein Verfahren zum Verfolgen einer chirurgischen Referenzierungsvorrichtung im Raum mit einem chirurgischen Navigationssystem.

Es sind Referenzierungsvorrichtungen der eingangs genannten Art bekannt, die insbesondere in der Knie- oder Hüftprothetik zum Einsatz kommen, um dem Operateur die Implantation eines künstlichen Kniegelenkes oder eines künstlichen Hüftgelenkes zu erleichtern. Die Referenzierungsvorrichtung wird an einem Körperteil festgelegt, dessen Bewegung im Raum mittels des Navigationssystems verfolgt werden soll. Anhand der Positionsdaten, die vom Navigationssystem beim Detektieren von von den Markerelementen stammender Strahlung (insbesondere elektromagnetische Strahlung und speziell Infrarotstrahlung) bestimmt werden, kann die Datenverarbeitungsvorrichtung die Lage und Orientierung der ihr bekannten Markerelementanordnung errechnen, woraus sie die Lage und Orientierung des zu verfolgenden Körperteils (insbesondere Femur oder Tibia) im Raum ermittelt.

Um eine zuverlässige Referenz zu erhalten, sind die Markiereinrichtungen als starre Körper (sogenannte "Rigid Bodies") ausgestaltet, die eine fest vorgegebene, dem Navigationssystem bekannte Relativanordnung der Markerelemente zueinander aufweisen. Zur Erzielung einer stabilen Referenz wird die Referenzierungsvorrichtung über die Befestigungseinrichtung üblicherweise über eine Knochenschraube, teilweise bikortikal, am Femur oder der Tibia verschraubt und dadurch fest an diesen fixiert. Dies führt jedoch zu einem Eingriff nicht unerheblicher Invasivität, gegenüber dem bei einigen Operateuren und Patienten Vorbehalte bestehen. Zur Verringerung der Invasivität ist es bekannt, Rigid Bodies an einer um den Femur oder die Tibia gelegten Manschette zu fixieren, was sich in der Praxis jedoch als nachteilig auf die Genauigkeit der Referenz erweist.

Ausführungsformen unterschiedlicher Referenzierungsvorrichtungen sind in den folgenden Druckschriften offenbart: EP 2 298 215 A1,
WO 2011/023232 A1, WO 2012/068679 A1, EP 1 518 508 A1,
WO 2011/113482 A1, EP 2 198 801 A1 und US 2004/0106861 A1.

Die EP 2 179 682 A1 beschreibt ein Patientenüberwachungssystem. Wenn von einem Positionserfassungsgerät festgestellt wird, dass von einem an einem Patienten angebrachten Emitter kein Signal empfangen wird, wird ein Warnsignal für einen Operateur ausgegeben.

Aufgabe der vorliegenden Erfindung ist es, eine gattungsgemäße Referenzierungsvorrichtung und ein gattungsgemäßes Navigationssystem bereitzustellen, die bzw. das eine geringere Invasivität bei der Befestigung der Referenzierungsvorrichtung am Körper eines Patienten ermöglichen.

Diese Aufgabe wird erfindungsgemäß durch eine Referenzierungsvorrichtung mit den Merkmalen von Anspruch 1 gelöst.

Bei der erfindungsgemäßen Referenzierungsvorrichtung ist die Möglichkeit gegeben, dass zwei oder mehr Markerelemente positionsveränderlich relativ zueinander sind und sich relativ zueinander bewegen können. Es wird zugelassen, dass sich die Geometrie der Markerelementanordnung ändert. Dadurch erfolgt eine Abkehr vom herkömmlichen Konzept des "Rigid Bodies" zur Definition einer Referenz, zu deren Zuverlässigkeit eine starre Markerelementanordnung gefordert ist, bei der die einzelnen Markerelemente starr miteinander verbunden sind und sich nicht relativ zueinander bewegen können. In die Erfindung fließt der Gedanke mit ein, dass bei einer nicht-starren Markerelementanordnung eine Beweglichkeit der Markerelemente relativ zueinander zugelassen werden kann, sofern die Relativbewegung der Markerelemente beim bestimmungsgemäßen Gebrauch der Referenzierungsvorrichtung mit dem Navigationssystem im Allgemeinen klein ist gegenüber der Absolutbewegung der Markerelementanordnung, die auf einer Bewegung der Referenzierungsvorrichtung bei der Bewegung des Körperteils beruht.

Mit der erfindungsgemäßen Referenzierungsvorrichtung ist es insbesondere möglich, das Körperteil weniger invasiv zu markieren, als dies beispielsweise bei einer Befestigung der Referenzierungsvorrichtung über eine Knochenschraube der Fall ist. Es ist vorzugsweise insbesondere die Möglichkeit gegeben, die Markerelemente über eine Manschette oder dergleichen am Patientenkörper zu befestigen. Gegenüber herkömmlichen "Rigid Bodies", welche ebenfalls mit einer Manschette am Körperteil befestigt werden, beruht der Vorteil der erfindungsgemäßen Referenzierungsvorrichtung allerdings auf der relativen Beweglichkeit der Markerelemente. Bei herkömmlichen Referenzierungsvorrichtungen mit starren Markerelementanordnungen kann nicht festgestellt werden, ob eine Bewegung der Markiereinrichtung im Raum auf einer Bewegung des Körperteils oder einer Bewegung der Markiereinrichtung relativ zum Körperteil aufgrund nicht-starrer Befestigung beruht. Unter Einsatz der erfindungsgemäßen Referenzierungsvorrichtung kann dies demgegenüber unter der Annahme festgestellt werden, dass die Relativbeweglichkeit der Markerelemente zueinander geringer ist als die Absolutbewegung der Markerelementanordnung im Raum infolge der Bewegung eines Körperteils.

Günstig ist es, dass die Befestigungseinrichtung einen einer Mehrzahl der Markerelemente zugeordneten Trägerkörper aufweist oder ausbildet, an dem die Markerelemente festlegbar sind. Über den Trägerkörper kann eine Mehrzahl von Markerelementen gemeinsam am Körper des Patienten befestigt werden.

Der Trägerkörper ist formveränderlich, wodurch er sich auf einfache Weise an die Anatomie des Patienten anpassen kann.

Von Vorteil ist es, dass Markerelemente lösbar am Trägerkörper festlegbar ist, beispielsweise über ein Befestigungselement, das eine lösbare Klebeschicht oder einen Saugnapf umfasst, oder über eine Klettverbindung mit Befestigungselementen in Gestalt eines Flausch- und eines Hakenbandes. Dadurch, dass ein Markerelement lösbar am Trägerkörper festlegbar ist, kann es repositioniert werden. Dies erlaubt es beispielsweise, die Genauigkeit der von der Markerelementanordnung gebildeten Referenz zu steigern, zum Beispiel indem deren Geometrie und/oder Größe in einer Weise verändert wird, die dem Navigationssystem es erleichtert, die Relativbewegung der Markerelemente zueinander von der Absolutbewegung der Referenzierungsvorrichtung zu unterscheiden.

Vorzugsweise sind Markerelemente getrennt voneinander positionierbar, insbesondere individuell am Körper des Patienten oder am Trägerkörper der Befestigungseinrichtung festlegbar. Dadurch erweist sich die Referenzierungsvorrichtung als besonders vielseitig. Die Markerelemente können so positioniert werden, dass sie von der Nachweisvorrichtung des Navigationssystems möglichst gut gesehen und eine möglichst genaue Referenz erzielt werden kann, selbst wenn die Markerelemente relativ zueinander positionsveränderlich sind. Als vorteilhaft erweist es sich, wenn die Markerelemente paarweise positionsveränderlich zueinander sind.

Es kann auch vorgesehen sein, dass ein Teil der Markerelemente relativ zueinander nicht-positionsveränderlich sind, wohingegen mindestens ein weiteres Markerelement positionsveränderlich zu den erstgenannten Markerelementen ist.

Bevorzugt sind die Markerelemente gruppenweise positionsveränderlich zueinander, wobei eine Gruppe von Markerelementen mindestens zwei Markerelemente umfasst und zwei oder mehr Gruppen von Markerelementen vorhanden sind.

Besonders günstig ist es, wenn die Befestigungseinrichtung so ausgebildet ist, dass die Markiereinrichtung nicht-invasiv am Körper des Patienten festlegbar ist. Als "nicht-invasiv" wird vorliegend insbesondere verstanden, dass die Markiereinrichtung ohne Inzision, speziell ohne gesonderte Inzision nur zur Festlegung der Markiereinrichtung, und/oder ohne Einsatz eines Knochenverankerungselementes am Körper des Patienten festlegbar ist.

Vorteilhafterweise umfasst die Befestigungseinrichtung an einem oder an mehreren Markerelementen ein Befestigungselement. Jedem Markerelement kann ein eigenes Befestigungselement zugeordnet sein, oder zwei oder mehr Markerelementen kann ein gemeinsames Befestigungselement zugeordnet sein. Die Befestigungselemente der Markerelemente können identisch oder unterschiedlich sein. Über das Befestigungselement ist eine Befestigung direkt am Patientenkörper möglich oder indirekt, beispielsweise über den erwähnten Trägerkörper.

Bei einer vorteilhaften Ausführungsform der erfindungsgemäßen Referenzierungsvorrichtung umfasst mindestens ein Befestigungselement eine Klebeschicht. Über die Klebeschicht kann das Markerelement beispielsweise direkt am Körper des Patienten oder am Trägerkörper klebend befestigt werden, der seinerseits am Körper des Patienten befestigt wird.

Alternativ oder ergänzend kann vorgesehen sein, dass mindestens ein Befestigungselement ein Flausch- oder Hakenband ist zum Zusammenwirken mit einem Haken- bzw. Flauschband am Trägerkörper der Befestigungseinrichtung. Durch Zusammenwirken eines Flauschbandes mit einem Hakenband kann eine Klettverbindung-Fixierung gebildet werden zur Befestigung des Markerelementes am Trägerkörper. Jedem Markerelement kann ein separates Flausch- oder Hakenband zugeordnet sein, oder mehreren Markerelementen ist ein gemeinsames Flausch- oder Hakenband zugeordnet.

Alternativ oder ergänzend kann vorgesehen sein, dass mindestens ein Befestigungselement ein chirurgisches Knochenverankerungselement ist oder umfasst. Mindestens ein Befestigungselement kann ein Knochenverankerungselement sein, beispielsweise eine Knochenschraube oder ein Kirschner-Draht. Bei den andersartigen Befestigungselementen handelt es sich vorzugsweise um ein Flausch- oder Hakenband, einen Saugnapf oder um ein Befestigungselement umfassend eine Klebeschicht. Ein Markerelement, das über ein Knochenverankerungselement an einem Knochen fixiert wird, kann zur Verbesserung der Genauigkeit eine besonders stabile Referenz am Körper des Patienten bilden. Diese Referenz kann vom Navigationssystem vertrauenswürdiger angesehen werden, wenn es darum geht, zur Ermittlung der Änderung der Lage und Orientierung der Markerelementanordnung eine Absolutbewegung der Markerelementanordnung von einer Relativbewegung der Markerelemente zueinander zu unterscheiden.

Günstig ist es, wenn die am Trägerkörper festlegbaren Markerelemente getrennt voneinander am und insbesondere auf dem Trägerkörper positionierbar und/oder individuell am Trägerkörper festlegbar sind, wodurch der Referenzierungsvorrichtung eine hohe Vielseitigkeit verliehen wird.

Es kann vorgesehen sein, dass mindestens ein Markerelement unlösbar am Trägerkörper festgelegt ist, beispielsweise mit diesem vernäht, auf diesen aufgeklebt oder mit diesem thermisch verschweißt.

Der Trägerkörper weist vorzugsweise ein Haken- oder Flauschband auf zum Zusammenwirken mit einem Flausch- bzw. einem Hakenband an einem oder mehreren Markerelementen. Wie bereits erwähnt, können ein oder mehr Markerelemente dadurch über eine Klettverbindung lösbar mit dem Trägerkörper verbunden werden.

Bei einer vorteilhaften Ausführungsform der erfindungsgemäßen Referenzierungsvorrichtung erweist es sich als vorteilhaft, wenn der Trägerkörper ein Textilverbund ist oder einen solchen umfasst. Der Textilverbund kann sich auf einfache Weise an die Anatomie des Patienten anpassen, wodurch die Referenzierungsvorrichtung eine hohe Vielseitigkeit aufweist. Insbesondere ist eine nicht-invasive Festlegung der Markiereinrichtung am Patientenkörper auf einfache Weise möglich.

Der Trägerkörper ist bei einer vorteilhaften Ausführungsform eine Bandage oder umfasst eine solche. Beispielsweise handelt es sich um eine umschnallbare und bevorzugt längenveränderliche Bandage, die sich auf einfache Weise an Körperteile unterschiedlicher Größe anpassen lässt.

Bei einer weiteren vorteilhaften Ausführungsform der Referenzierungsvorrichtung ist der Trägerkörper günstigerweise ein Schlauch- oder Strumpfkörper oder umfasst einen solchen. Der Trägerkörper kann insbesondere ringförmig sein, schlauch- oder strumpfartig über das Körperteil gestreift und dadurch an diesem festgelegt werden. Zur Anpassung an den Patienten weist der Trägerkörper eine verformbare, beispielsweise gummielastische Ausgestaltung auf.

Bei einer konstruktiv einfachen Ausgestaltung der Referenzierungsvorrichtung ist es günstig, wenn der Trägerkörper auf dem Patientenkörper aufklebbar ist. Durch Ausgestaltung des Trägerkörpers als Aufkleber kann die Referenzierungsvorrichtung besonders einfach ausgestaltet und am Körper des Patienten befestigt werden.

Insbesondere bei der zuletzt erwähnten vorteilhaften Ausführungsform ist es günstig, wenn der Trägerkörper eine folienförmige Ausgestaltung aufweist.

Bei einer weiteren vorteilhaften Ausführungsform der erfindungsgemäßen Referenzierungsvorrichtung umfasst der Trägerkörper mindestens einen Hohlraum oder bildet einen solchen aus und mindestens ein Anschlusselement für eine Saug- und/oder Druckleitung, wobei der mindestens eine Hohlraum mit Unter- oder Überdruck beaufschlagbar ist. Über das mindestens eine Anschlusselement kann der mindestens eine Hohlraum mit Unter- oder Überdruck beaufschlagt werden, wodurch die Formstabilität des Trägerkörpers gesteigert werden kann. Beispielsweise kommt diese Ausgestaltung bei einem Trägerkörper in Form einer Bandage, einer Manschette oder eines Schlauch- oder Strumpfkörpers zum Einsatz, wobei der Trägerkörper handhabungsfreundlich unter Veränderung der Form am Patientenkörper festgelegt werden kann. Bei nachfolgender Beaufschlagung mit Unter- oder Überdruck kann die Formstabilität des Trägerkörpers gesteigert werden. Dadurch lässt sich beispielsweise die Beweglichkeit der Markerelemente relativ zueinander verringern und der Trägerkörper zugleich zuverlässiger am Patientenkörper fixieren.

Es kann vorgesehen sein, dass die Befestigungseinrichtung an einem oder mehreren Markerelementen ein Befestigungselement umfasst und/oder mindestens ein am Trägerkörper angeordnetes Befestigungselement, welches Befestigungselement ein Saugnapf ist oder einen solchen umfasst. Über den Saugnapf kann das Markerelement (am Trägerkörper oder direkt am Patientenkörper) oder der Trägerkörper am Patientenkörper handhabungsfreundlich lösbar festgelegt werden.

Der Trägerkörper kann auf seiner im bestimmungsgemäßen Gebrauch der Referenzierungsvorrichtung zugewandten Seite reibwertsteigernde Elemente umfassen, mit denen eine möglichst große Reibung zwischen dem Trägerkörper und dem Körper des Patienten erzielt werden kann. Beispielsweise sind punkt- oder streifenförmige reibwertsteigernde Elemente vorgesehen, insbesondere aus einem Silikonmaterial.

Zur Erzielung einer konstruktiv einfachen Ausgestaltung ist es günstig, wenn die Markerelemente identisch sind.

Wie bereits erwähnt, betrifft die Erfindung auch ein chirurgisches Navigationssystem. Die eingangs gestellte Aufgabe wird bei einem gattungsgemäßen chirurgischen Navigationssystem erfindungsgemäß dadurch gelöst, dass die Referenzierungsvorrichtung eine Referenzierungsvorrichtung der vorstehend beschriebenen Art ist.

Beim erfindungsgemäßen Navigationssystem ist es insbesondere möglich festzustellen, ob eine Bewegung der Markerelemente auf eine Bewegung des Körperteils zurückführbar ist, an dem die Referenzierungsvorrichtung befestigt ist, oder auf eine Bewegung der Markerelemente innerhalb der Markerelementanordnung, bei der sich die Markerelemente relativ zueinander bewegen können. In Bezug auf die Relativbeweglichkeit der Markerelemente zueinander innerhalb der Markerelementanordnung wird von der Datenverarbeitungsvorrichtung insbesondere angenommen, dass diese bei Einsatz des Navigationssystems im Allgemeinen geringer ist als eine Absolutbewegung der Referenzierungsvorrichtung im Raum.

Die bereits im Zusammenhang mit der Erläuterung der erfindungsgemäßen Referenzierungsvorrichtung erzielbaren Vorteile können unter Einsatz des Navigationssystems ebenfalls erzielt werden, so dass diesbezüglich zur Vermeidung von Wiederholungen auf vorangegangene Ausführungen verwiesen werden kann. Weiter wird verwiesen auf die Erläuterungen vorteilhafter Ausführungsformen der Referenzierungsvorrichtung und der damit erzielbaren Vorteile.

Günstig ist es, wenn die Datenverarbeitungsvorrichtung so ausgebildet und programmiert ist, dass sie die Geometrie der Markerelementanordnung anhand der Positionsdaten ermittelt, wenn die Referenzierungsvorrichtung im Raum bewegt wird. Die Lage der einzelnen Markerelemente zueinander, oder deren Relativorientierung, definiert die Geometrie der Markerelementanordnung. Die Datenverarbeitungsvorrichtung kann diese gewissermaßen "automatisch" ermitteln, ohne dass sie der Datenverarbeitungsvorrichtung bekannt sein muss, wie dies bei herkömmlichen Referenzierungsvorrichtungen der Fall ist. Dadurch ist insbesondere auch die Möglichkeit gegeben, eine Referenzierungsvorrichtung ohne vorheriges Wissen über ihr Vorhandensein selbsttätig zu identifizieren.

Es kann vorgesehen sein, dass an einer Hinweiseinheit des Navigationssystems ein Hinweis für den Benutzer ausgegeben wird, das Körperteil des Patienten mit der daran befestigten Referenzierungsvorrichtung im Raum zu bewegen. Anhand der Positionsdaten kann die Datenverarbeitungsvorrichtung Anzahl und Relativorientierung der Markerelemente und damit die Geometrie der Markerelementanordnung analysieren, ermitteln und speichern, um die Markerelementanordnung für die Verfolgung der Referenzierungsvorrichtung im Raum zugrunde zu legen.

Vorzugsweise kann die Datenverarbeitungsvorrichtung so ausgebildet und programmiert sein, dass sie durch Analyse der Positionsdaten bei der Verfolgung der Referenzierungsvorrichtung im Raum ebenfalls automatisch eine Änderung der Markerelementanordnung feststellt. Die gespeicherte Geometrie kann erforderlichenfalls durch die neu festgestellte Geometrie der Markerelementanordnung ersetzt werden.

Vorteilhafterweise ist die Datenverarbeitungsvorrichtung so ausgebildet und programmiert, dass sie ermittelt, welche Eigenbeweglichkeit die Markerelemente innerhalb der Markerelementanordnung aufweisen, und dass sie in einer Speichereinheit die jeweilige Eigenbeweglichkeit speichert oder eine bereits gespeicherte Eigenbeweglichkeit aktualisiert und bei einer nachfolgenden Ermittlung der Änderung der Lage und der Orientierung der Referenzierungsvorrichtung im Raum zugrunde legt. Dies erfolgt vorzugsweise bei dem anfänglichen Ermitteln der Geometrie der Markerelementanordnung wie vorstehend beschrieben oder im Anschluss daran, beispielsweise noch bevor der eigentliche Eingriff durchgeführt wird. Möglich ist es allerdings auch, dass dies während des Eingriffs bei der Verfolgung der Referenzierungsvorrichtung im Raum durchgeführt wird. Die jeweilige Eigenbeweglichkeit eines Markerelementes kann insbesondere von der Datenverarbeitungsvorrichtung laufend überwacht und aktualisiert werden.

Als Eigenbeweglichkeit eines Markerelementes wird vorliegend insbesondere dessen Möglichkeit gesehen, sich innerhalb der Markerelementanordnung relativ zu den anderen Markerelementen zu bewegen. Eine derartige Bewegung kann beispielsweise auf die Verformung des Körperteils zurückzuführen sein, an dem die Referenzierungsvorrichtung festgelegt ist, auf eine Bewegung oder Verformung des Trägerkörpers, an dem das Markerelement festgelegt ist oder auf eine Bewegung des Markerelementes relativ zum Trägerkörper bei nicht-starrer Verbindung mit demselben.

Dadurch, dass der Datenverarbeitungsvorrichtung die Eigenbeweglichkeit der Markerelemente bekannt ist, kann sie bei einer Verfolgung der Referenzierungsvorrichtung eine Bewegung der Markerelemente, die zu einer Änderung der Geometrie der Markerelementanordnung führt, kompensieren. Dementsprechend kann die Datenverarbeitungsvorrichtung ermitteln, ob sich ein Markerelement innerhalb der Eigenbeweglichkeit und damit gewissermaßen "innerhalb erlaubter Grenzen" bewegt hat. Innerhalb dieser Grenzen sieht die Datenverarbeitungsvorrichtung die Position des Markerelementes innerhalb der Markerelementanordnung als korrekt an.

Vorzugsweise ist die Datenverarbeitungsvorrichtung so ausgebildet und programmiert, dass sie bei der Ermittlung der Eigenbeweglichkeit der Markerelemente berücksichtigt, um welchen Betrag und/oder in welcher Raumrichtung das jeweilige Markerelement innerhalb der Markerelementanordnung in Bezug auf die anderen Markerelemente der Markiereinrichtung bewegbar ist. Beispielsweise definiert die Datenverarbeitungsvorrichtung ein Ellipsoid für die jeweilige Beweglichkeit eines Markerelementes mit drei paarweise senkrecht aufeinander stehenden Achsen, deren Betrag jeweils die Fähigkeit eines Markerelementes angibt, sich längs der entsprechenden Achse innerhalb der Markerelementanordnung bewegen zu können.

Günstig ist es, wenn die Datenverarbeitungsvorrichtung so ausgebildet und programmiert ist, dass sie Positionsdaten von Markerelementen abhängig von der Größe der jeweiligen Eigenbeweglichkeit bei einer Ermittlung der Änderung der Lage und der Orientierung der Referenzierungsvorrichtung im Raum unterschiedlich gewichtet, wobei Markerelemente mit geringer Eigenbeweglichkeit eine höhere Gewichtung erhalten als Markerelemente größerer Eigenbeweglichkeit. Die Datenverarbeitungsvorrichtung kann Markerelemente verhältnismäßig geringer Eigenbeweglichkeit als vertrauenswürdiger ansehen als Markerelemente verhältnismäßig großer Eigenbeweglichkeit. Dies erlaubt es der Datenverarbeitungsvorrichtung, ein Maß für die Bestimmtheit oder Genauigkeit von Positionsdaten zu ermitteln, die auf ein Markerelement zurückzuführen sind. Dementsprechend kann die Datenverarbeitungsvorrichtung bei der Verfolgung der Referenzierungsvorrichtung unter Bestimmung der Markerelementanordnung Positionsdaten eines vertrauenswürdigen Markerelementes stärker gewichten als Positionsdaten eines weniger vertrauenswürdigen Markerelementes. Dadurch kann auch ein Maß für die Bestimmtheit und Genauigkeit festgestellt werden, mit der die Markerelementanordnung erkannt wird.

Von Vorteil ist es, wenn die Datenverarbeitungsvorrichtung so ausgebildet und programmiert ist, dass sie unter Berücksichtigung der Eigenbeweglichkeiten der Markerelemente die Genauigkeit einschätzt, eine Änderung der Lage und der Orientierung der Referenzierungsvorrichtung im Raum ermitteln zu können, und wenn die Datenverarbeitungsvorrichtung bei nicht hinreichender Genauigkeit einen Hinweis an einer Hinweiseinheit des Navigationssystems vorschlagend die Änderung der Position mindestens eines Markerelementes oder die Hinzufügung mindestens eines weiteren Markerelementes zur Markerelementanordnung ausgibt. Mit dem Ziel, eine möglichst genaue Referenz mit der Referenzierungsvorrichtung zu erzielen, kann die Datenverarbeitungsvorrichtung vorzugsweise bereits beim anfänglichen Erkennen und Identifizieren der Markerelementanordnung und der Eigenbeweglichkeiten der Markerelemente einen oder mehrere Hinweise an der Hinweiseinheit (beispielsweise einem Bildschirm) ausgeben. Durch Änderung der Position eines oder mehrerer Markerelemente oder Hinzufügung mindestens eines Markerelementes kann das Maß für die Genauigkeit, mit der die Referenzierungsvorrichtung im Raum verfolgt werden kann, gesteigert werden.

Beispielsweise ist die Datenverarbeitungsvorrichtung so ausgebildet und programmiert, dass sie einen Hinweis betreffend die Änderung der Position eines Markerelementes ausgibt, dessen Eigenbeweglichkeit eine Schwellenwertbeweglichkeit übersteigt.

Günstigerweise ist die Datenverarbeitungsvorrichtung so ausgebildet und programmiert, dass sie bei der Ermittlung der Änderung der Lage und der Orientierung der Referenzierungsvorrichtung im Raum ermittelt, ob eine Bewegung eines jeweiligen Markerelementes innerhalb der Markerelementanordnung eine Schwellenwertbewegung überschreitet. Wird die Referenzierungsvorrichtung im Raum verfolgt, kann die Datenverarbeitungsvorrichtung anhand der Positionsdaten feststellen, ob sich das Markerelement in einer Weise innerhalb der Markerelementanordnung relativ zu den anderen Markerelementen bewegt, die eine Schwellenwertbewegung übersteigt. Beispielsweise wird überprüft, ob die Bewegung des Markerelementes innerhalb der Eigenbeweglichkeit liegt, insbesondere innerhalb des vorstehend erwähnten Beweglichkeitsellipsoides des Markerelementes.

Von Vorteil ist es, wenn die Datenverarbeitungsvorrichtung so ausgebildet und programmiert ist, dass sie bejahendenfalls Positionsdaten des jeweiligen Markerelementes weniger stark bei der Ermittlung der Änderung der Lage und Orientierung der Referenzierungsvorrichtung im Raum gewichtet als Positionsdaten anderer Markerelemente, bei denen die Bewegung innerhalb der Markerelementanordnung geringer ist. Dies gibt, wie bereits erwähnt, die Möglichkeit, Markerelemente bei der Verfolgung der Referenzierungsvorrichtung als unterschiedlich vertrauenswürdig anzusehen. Markerelemente geringerer Bewegung innerhalb der Markerelementanordnung können zur Verfolgung der Referenzierungsvorrichtung höher gewichtet werden als Markerelemente, deren Bewegung die Schwellenwertbewegung überschreitet.

Vorteilhafterweise ist die Datenverarbeitungsvorrichtung so ausgebildet und programmiert, dass sie die Positionsdaten des jeweiligen Markerelementes bei der Ermittlung der Änderung der Lage und der Orientierung der Referenzierungsvorrichtung im Raum ignoriert, sofern die Datenverarbeitungsvorrichtung anhand der nicht-ignorierten Positionsdaten der übrigen Markerelemente eindeutig die Lage und die Orientierung der Referenzierungsvorrichtung im Raum ermitteln kann. Das Markerelement, dessen Bewegung die Schwellenwertbewegung überschreitet, kann bei der Bestimmung der Markerelementanordnung ignoriert werden, sofern anhand der Positionsdaten der übrigen Markerelemente eine eindeutige Bestimmung derselben möglich ist. Gewissermaßen werden nur die als "valide" von der Datenverarbeitungsvorrichtung angesehenen Markerelemente herangezogen, um die Markerelementanordnung zu bestimmen.

In entsprechender Weise kann vorgesehen sein, dass ein Markerelement außerhalb des Sichtfeldes der Nachweisvorrichtung ignoriert wird, d. h. wenn die Datenverarbeitungsvorrichtung feststellt, dass zu dem entsprechenden Markerelement keine Positionsdaten vorliegen. Die Datenverarbeitungsvorrichtung kann dann eine Identifizierung der Markerelementanordnung anhand der Positionsdaten der übrigen Markerelemente vornehmen, sofern dies eindeutig möglich ist.

Es kann vorgesehen sein, dass der Datenverarbeitungsvorrichtung die Lage einer körpereigenen Struktur oder einer durch diese definierten Achse, Fläche oder Ebene im Raum bekannt ist. Beispielsweise ist die Lage einer Ebene, etwa der Beckeneingangsebene, oder die Lage einer Körperachse, beispielsweise der mechanischen Femurachse oder der Tibiaachse, im Raum bekannt. Dies ermöglicht es, einen körperfesten Bezug der Referenzierungsvorrichtung ausnutzen. Eine Zielgeometrie wie die körpereigene Struktur, die Achse, Fläche oder Ebene kann mit der Verfolgung der Referenzierungsvorrichtung im Raum ebenfalls verfolgt werden.

Es ist insbesondere günstig, wenn die Datenverarbeitungsvorrichtung so ausgebildet und programmiert ist, dass sie bei Kenntnis der Lage der Referenzierungsvorrichtung relativ zu einer körpereigenen Struktur oder durch diese definierten Achse, Fläche oder Ebene des Patienten die Genauigkeit einschätzt, eine Änderung der Lage der körpereigenen Struktur, Achse, Fläche oder Ebene im Raum ermitteln zu können und dass die Datenverarbeitungsvorrichtung bei nicht hinreichender Genauigkeit einen Hinweis an einer Hinweiseinheit des Navigationssystems vorschlagend die Änderung der Position mindestens eines Markerelementes oder die Hinzufügung mindestens eines weiteren Markerelementes zur Markerelementanordnung ausgibt. Dadurch ist die Möglichkeit gegeben, die Genauigkeit hinsichtlich der körpereigenen Struktur, Achse, Fläche oder Ebene zu steigern. Eine möglichst genaue Verfolgung der körpereigenen Struktur, Achse, Fläche oder Ebene anhand der Referenzierungsvorrichtung ist für die Kenntnis der anatomischen Gegebenheiten des Patienten von Vorteil, beispielsweise bei einer Hüftoperation oder einer Knieoperation.

Von Vorteil ist es, wenn die Datenverarbeitungsvorrichtung so ausgebildet und programmiert ist, dass sie an einer Hinweiseinheit des Navigationssystems, beispielsweise an einem Bildschirm und bevorzugt vor dem eigentlichen Eingriff, einen Hinweis für den Benutzer ausgibt betreffend die Anzahl und/oder die Position der einzusetzenden Markerelemente bei deren Anordnung am Körper des Patienten, insbesondere den Abstand der Markerelemente voneinander und/oder deren Anordnung relativ zueinander und/oder einen Hinweis betreffend die Art und Weise der Befestigung der Markerelemente am Körper des Patienten über die Befestigungseinrichtung. Beispielsweise Anzahl der Markerelemente, Abstand voneinander, Relativposition, Art des Körperteils, und/oder Art der Befestigung können vom Navigationssystem vorgeschlagen und dadurch der Operateur bei der Anordnung der Referenzierungsvorrichtung angeleitet und unterstützt werden. Dies erfolgt insbesondere mit dem Ziel, eine möglichst genaue Referenz mit der Referenzierungsvorrichtung zu erzielen.

Als günstig erweist es sich, wenn das Navigationssystem eine Speichereinheit aufweist, in der Muster für Markerelementanordnungen hinsichtlich der Anzahl und/oder Relativorientierung der Markerelemente, deren Position der Festlegung am Körper des Patienten und/oder hinsichtlich der Art und Weise der Befestigung der Markerelemente am Körper des Patienten über die Befestigungseinrichtung gespeichert sind und wenn die Datenverarbeitungsvorrichtung so ausgebildet und programmiert ist, dass sie den Hinweis unter Berücksichtigung mindestens eines aus der Speichereinheit gelesenen Musters ausgibt. Anhand der Muster oder "Templates" kann ein Hinweis an den Benutzer ausgegeben werden. Basierend auf Erfahrungswerten und/oder Vorwissen kann der Operateur dadurch besonders gezielt bei der Positionierung und/oder Befestigung der Markerelemente angeleitet und unterstützt werden.

Wie eingangs erwähnt betrifft die Erfindung auch ein Verfahren. Ein erfindungsgemäßes Verfahren, mit dem die eingangs gestellte Aufgabe ebenfalls gelöst werden kann, ist vorgesehen zum Verfolgen einer chirurgischen Referenzierungsvorrichtung im Raum mit einem chirurgischen Navigationssystem der vorstehend genannten Art, welches eine Referenzierungsvorrichtung der vorstehend genannten Art umfasst, wobei eine Nachweisvorrichtung des Navigationssystems von den Markerelementen reflektierte

Strahlung detektiert und diesbezügliche Positionsdaten bereitstellt und eine Datenverarbeitungsvorrichtung des Navigationssystems die Positionsdaten zum Ermitteln der Lage und der Orientierung der Referenzierungsvorrichtung im Raum verarbeitet, wobei die Datenverarbeitungsvorrichtung eine Änderung der Lage und Orientierung der Referenzierungsvorrichtung im Raum ermittelt.

Die bereits im Zusammenhang mit der erfindungsgemäßen Referenzierungsvorrichtung und dem erfindungsgemäßen Navigationssystem erzielbaren Vorteile können unter Einsatz des Verfahrens erzielt werden. Zur Vermeidung von Wiederholungen, auch in Bezug auf die jeweiligen Ausführungsformen, wird daher auf voranstehende Ausführungen verwiesen. Vorteilhafte Ausführungsformen der Referenzierungsvorrichtung und des Navigationssystems können herangezogen werden, um vorteilhafte Ausführungsbeispiele des Verfahrens zu bilden, so dass diesbezüglich ebenfalls auf voranstehende Ausführungen verwiesen wird.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Ansicht eines erfindungsgemäßen Navigationssystems bei Anwendung an einem Patienten, umfassend eine vorteilhafte Ausführungsform einer erfindungsgemäßen Referenzierungsvorrichtung, wobei das Navigationssystem zur Durchführung eines vorteilhaften Ausführungsbeispiels des erfindungsgemäßen Verfahrens ausgebildet ist;
- Figur 2:: eine vergrößerte Darstellung von Detail A in Figur 1;
- Figur 3:: eine perspektivische Darstellung der Referenzierungsvorrichtung des Navigationssystems aus Figur 1 (im geöffneten Zustand) sowie ein Bein des Patienten;
- Figur 4:: ein Markerelement der Referenzierungsvorrichtung vor Festlegung an einem Trägerkörper der Referenzierungsvorrichtung (teilweise);
- Figur 5:: das Markerelement, am Trägerkörper festgelegt (teilweise);
- Figur 6:: eine Darstellung entsprechend Figur 3 bei einer zweiten vorteilhaften Ausführungsform einer erfindungsgemäßen Referenzierungsvorrichtung;
- Figur 7:: eine Darstellung entsprechend Figur 3 bei einer dritten vorteilhaften Ausführungsform einer erfindungsgemäßen Referenzierungsvorrichtung;
- Figur 8:: die Referenzierungsvorrichtung aus Figur 7 in einer Seitenansicht (teilweise);
- Figur 9:: eine Darstellung entsprechend Figur 3 bei einer vierten, nicht beanspruchten, vorteilhaften Ausführungsform einer Referenzierungsvorrichtung;
- Figur 10:: eine Seitenansicht der Referenzierungsvorrichtung aus Figur 9 (teilweise);
- Figur 11:: eine schematische Seitenansicht bei einer fünften, erfindungsgemäßen, vorteilhaften Ausführungsform einer Referenzierungsvorrichtung;
- Figur 12:: eine schematische Seitenansicht einer sechsten, nicht beanspruchten, vorteilhaften Ausführungsform einer Referenzierungsvorrichtung und
- Figur 13:: eine schematische Draufsicht auf einen Trägerkörper einer erfindungsgemäßen Referenzierungsvorrichtung.

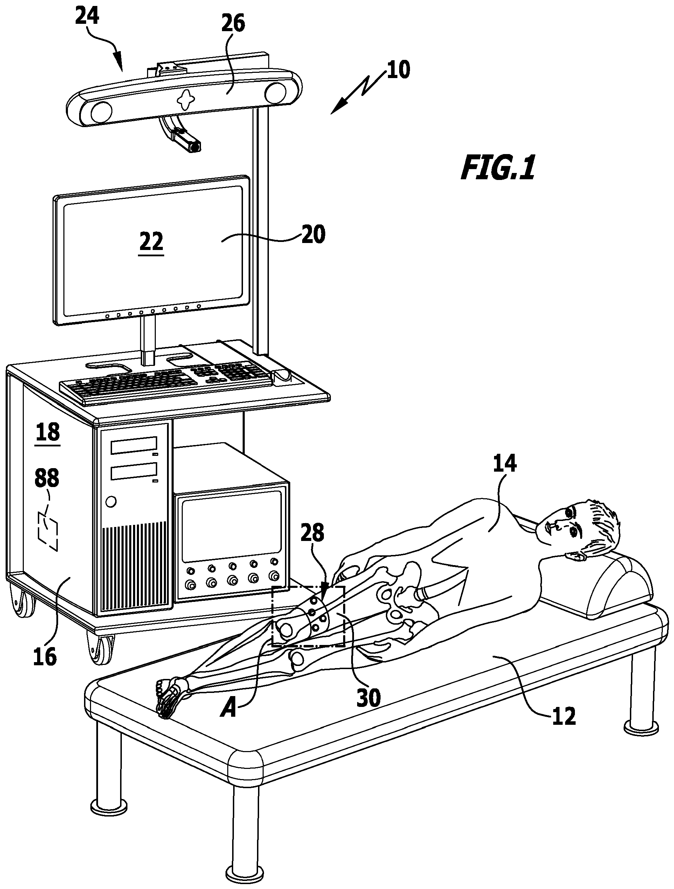

Figur 1 zeigt in perspektivischer Darstellung eine mit dem Bezugszeichen 10 belegte vorteilhafte Ausführungsform eines erfindungsgemäßen Navigationssystems sowie einen auf einem Operationstisch 12 liegend positionierten Patienten 14.

Das Navigationssystem 10 umfasst eine Datenverarbeitungsvorrichtung 16 in Gestalt eines Computers 18 und eine an diesen angeschlossene Hinweiseinheit 20 in Gestalt eines Bildschirms 22. Weiter umfasst das Navigationssystem 10 eine Nachweisvorrichtung 24, die insbesondere eine Stereokamera 26 aufweist. Mit der Stereokamera 26 kann elektromagnetische Strahlung, insbesondere Infrarotstrahlung, erfasst und ein diesbezügliches Signal an die Datenverarbeitungsvorrichtung 16 übermittelt werden. Von der Stereokamera 26 kann elektromagnetische Strahlung auch emittiert werden, die von einer Referenzierungsvorrichtung des Navigationssystems 10 reflektiert und daraufhin wieder von der Stereokamera 26 erfasst wird.

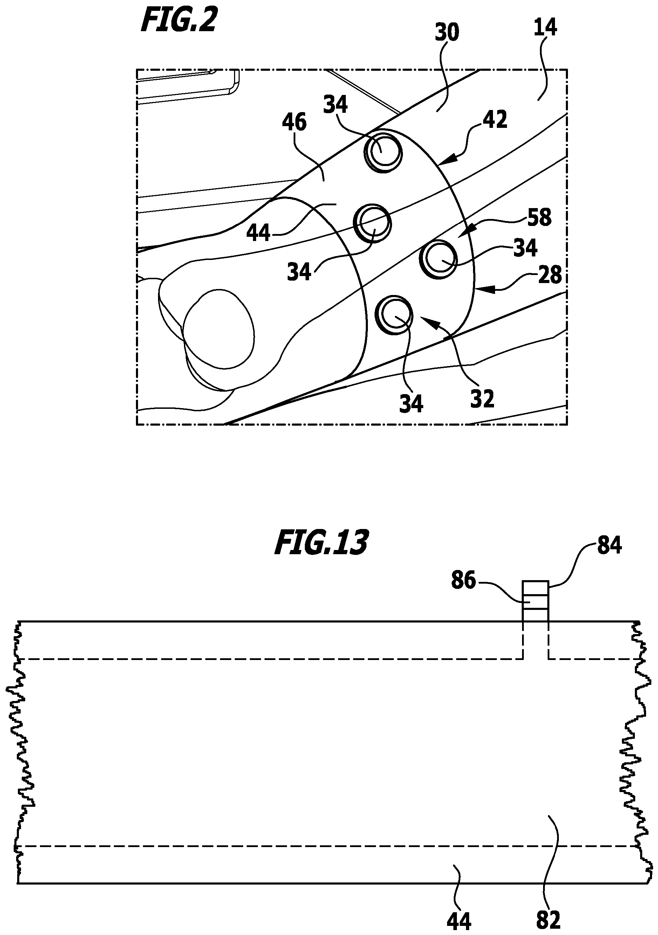

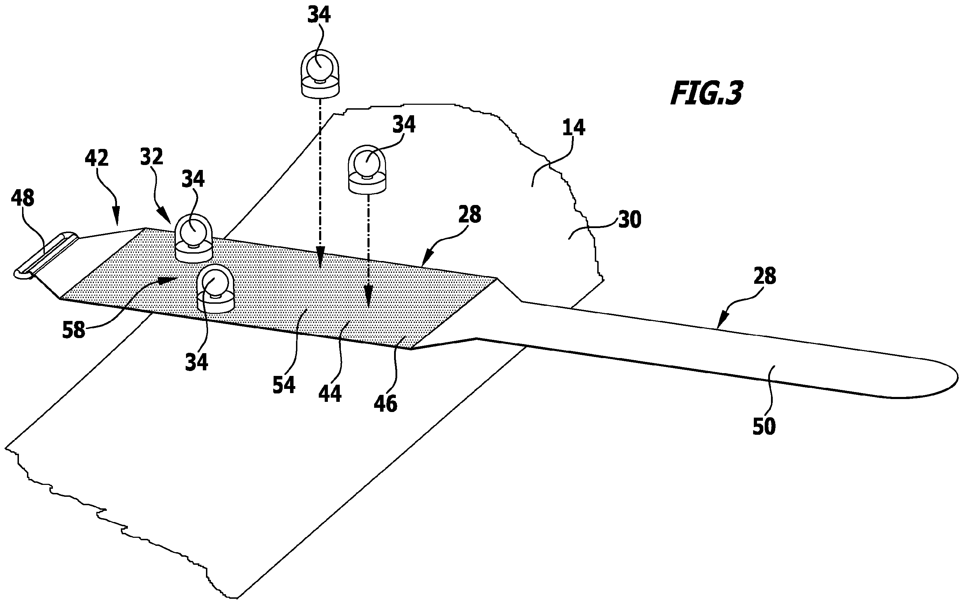

Figur 1 zeigt eine mit dem Bezugszeichen 28 belegte vorteilhafte Ausführungsform einer erfindungsgemäßen Referenzierungsvorrichtung, die in Figur 2 vergrößert und in Figur 3 detaillierter dargestellt ist. Die Referenzierungsvorrichtung 28 ist vorliegend an einem Körperteil des Patienten 14 befestigt, nämlich dem Oberschenkel 30, beispielsweise nahe dem Knie. Bewegt sich der Oberschenkel 30 im Raum, zum Beispiel während einer Knie- oder Hüftoperation, bewegt sich auch die Referenzierungsvorrichtung 28 im Raum. Sie definiert eine Referenz, anhand derer das Navigationssystem 10 eine Bewegung der Referenzierungsvorrichtung 28 und damit des Oberschenkels 30 im Raum ermitteln kann. Insbesondere kann die Lage und/oder die Orientierung der Referenzierungsvorrichtung 28 und des Oberschenkels 30 vom Computer 18 ermittelt werden, worauf nachfolgend noch eingegangen wird.

Die Referenzierungsvorrichtung 28 umfasst eine chirurgische Markiereinrichtung 32, die eine Mehrzahl von Markerelementen 34 aufweist. Die Markerelemente 34 sind identisch ausgestaltet. Vorliegend sind insgesamt vier Markerelemente 34 vorhanden.

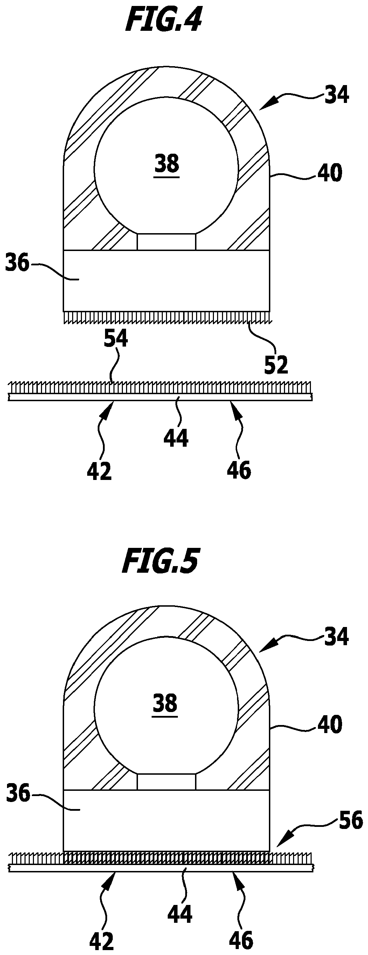

Der Aufbau der Markerelemente 34 geht aus den schematischen Darstellungen der Figuren 3 bis 5 hervor. Jedes Markerelement 34 umfasst ein Grundelement 36. Das Grundelement 36 bildet eine Basis oder einen Sockel für ein reflektierendes Element 38, das die elektromagnetische Strahlung der Stereokamera 26 reflektiert. Zum Schutz des reflektierenden Elementes 38 weist das Markerelement 34 ein haubenförmiges Schutzelement 40 auf, das das reflektierende Element 38 und das Grundelement 36 überdeckt und transparent für die elektromagnetische Strahlung der Stereokamera 26 ist.

Die Referenzierungsvorrichtung 28 umfasst zum Befestigen der Markiereinrichtung 32 am Patienten 14 eine Befestigungseinrichtung 42. Die Befestigungseinrichtung 42 weist einen Trägerkörper 44 auf, der am Patienten 14 lösbar befestigbar ist. Der Trägerkörper 44 ist vorliegend allen Markerelementen 34 zugeordnet, die wie nachfolgend erläutert auf diesem festlegbar sind.

Bei der Referenzierungsvorrichtung 28 ist der Trägerkörper 44 eine Bandage 46. Die Bandage 46 kann beispielsweise aus einem Textilverbund gefertigt sein oder aus einem gummielastischen Material. Die Bandage 46 kann von einem offenen, längserstreckten Zustand (Figur 3) in einen ringförmigen Zustand überführt werden, in welchem sie ringförmig um den Oberschenkel 30 geführt ist.

Die Bandage 46 ist längenveränderlich und kann in sich fixiert werden. Zu diesem Zweck weist sie an einer ersten Seite eine Öse 48 auf, durch die hindurch ein Bandagenabschnitt 50 an der gegenüberliegenden Seite der Bandage 46 geführt und dadurch die Bandage 46 festgezurrt werden kann. Alternativ kann beispielsweise eine Fixierung der Bandage 46 in sich mittels einer Schnalle, Druckknöpfen oder eines Klettverschlusses erfolgen (nicht gezeigt).

Die Bandage 46 ist verformbar, wodurch sie sich sehr gut an die Anatomie des Patienten 14 anpassen kann. Durch die zusätzliche Möglichkeit, die Länge der Bandage mittels des Bandagenabschnitts 50 und der Öse 48 einzustellen, erweist sich die Bandage 46 und damit die Referenzierungsvorrichtung 28 als besonders vielseitig.

An der im bestimmungsgemäßen Gebrauch den Patienten 14 zugewandten Seite kann der Trägerkörper 44 reibwertsteigernde Elemente aufweisen, beispielsweise Noppen oder Rippen aus einem Silikonmaterial. Zusätzlich zur Fixierung der Bandage 46 in sich ist dadurch die Möglichkeit gegeben, die Bandage 46 zuverlässig am Patienten 14 zu fixieren.

Die Befestigungseinrichtung 42 umfasst ferner zusammenwirkende Befestigungselemente 52, 54.

Jedem Markerelement 34 ist ein Befestigungselement 52 zugeordnet, das am jeweiligen Grundelement 36 festgelegt ist. Die Befestigungselemente 52 sind Flausch- oder Hakenbänder. Das Befestigungselement 54 ist ein Haken- oder Flauschband am Trägerkörper 44 und kann mit dem Flausch- bzw. Hakenband am jeweiligen Markerelement 34 zusammenwirken.

Infolgedessen wird durch die Befestigungselemente 52, 54 eine Klettverbindung 56 gebildet. Über die Klettverbindung 56 können die Markerelemente 34 lösbar am Trägerkörper 44 festgelegt werden. Jedes Markerelement 34 kann individuell am Trägerkörper 44 festgelegt und wieder von diesem gelöst werden, wodurch die Markerelemente getrennt voneinander positionierbar und über die Befestigungseinrichtung 42 getrennt voneinander am Patienten 14 festlegbar sind.

Die Markerelemente 34 bilden in Kombination miteinander eine Markerelementanordnung 58. Die Markerelementanordnung 58 ist hinsichtlich ihrer Geometrie veränderbar, da die Markerelemente 34 nicht starr miteinander verbunden, sondern individuell auf den Trägerkörper 44 fixierbar sind. Die Geometrie der Markerelementanordnung 58 kann sich auch dadurch ändern, dass sich die Bandage 46 verformt.

Da die Markerelemente 34 individuell auf der Bandage 46 festgelegt sind, kann dies zu einer Relativbewegung der Markerelemente 34 zueinander führen und infolgedessen zu einer Änderung der Geometrie der Markerelementanordnung 58.

Dabei ist es möglich, dass die Beweglichkeit der Markerelemente 34 unterschiedlich ist. Ein oder mehrere Markerelement(e) 34 kann/können eine höhere Beweglichkeit innerhalb der Markerelementanordnung 58 aufweisen als ein anderes oder mehrere andere Markerelemente 34. Umgekehrt ist es möglich, dass ein oder mehrere Markerelement(e) 34 eine geringere Beweglichkeit innerhalb der Markerelementanordnung 58 aufweist/aufweisen als ein anderes oder mehrere andere Markerelemente 34.

Die Geometrie der Markerelementanordnung 58 kann auch dadurch verändert werden, dass mindestens ein Markerelement 34 am Trägerkörper 44 repositioniert wird. Aufgrund der lösbaren Verbindung über die Klettverbindung 56 kann ein Benutzer mindestens ein Markerelement 34 vom Trägerkörper 44 lösen und an diesem unterschiedlich positioniert wieder festlegen.

Die Markerelementanordnung 58 kann auch dadurch verändert werden, dass ein Markerelement 34 gänzlich entfernt oder mindestens ein weiteres Markerelement 34 der Markiereinrichtung 32 hinzugefügt wird.

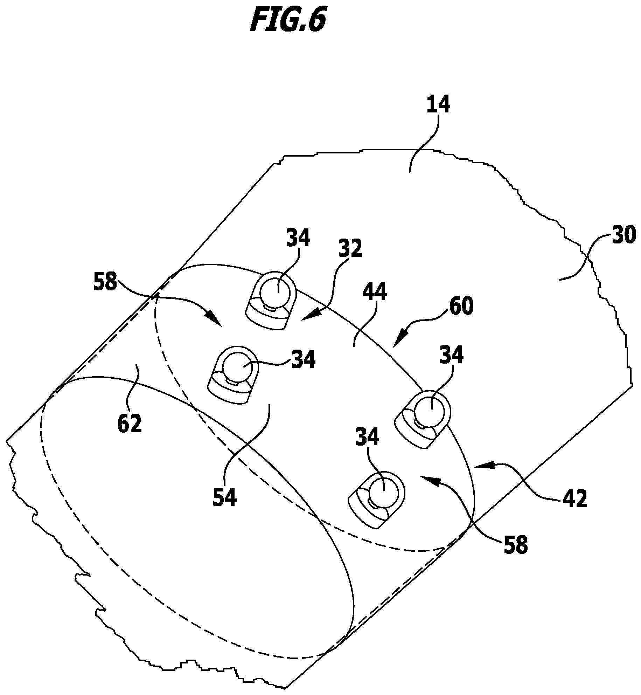

Nachfolgend wird unter Verweis auf die Figuren 6 bis 12 auf weitere vorteilhafte Ausführungsformen der erfindungsgemäßen Referenzierungsvorrichtung eingegangen. Für gleiche oder gleichwirkende Merkmale oder Komponenten der jeweiligen Referenzierungsvorrichtung werden identische Bezugszeichen verwendet.

Figur 6 zeigt eine Referenzierungsvorrichtung 60 mit vier Markerelementen 34. Die Befestigungseinrichtung 42 umfasst einen Trägerkörper 44, der ausgestaltet ist als in sich geschlossener ringförmiger Schlauchkörper 62 (oder Strumpfkörper 62).

Der Schlauchkörper 62 ist beispielsweise als Textilverbund gefertigt oder aus einem gummielastischen Material. Insbesondere ist der Schlauchkörper 62 verformbar, um sich an die Anatomie des Patienten 14 anzupassen. Auf der dem Patienten 14 zugewandten Seite können ebenfalls reibwertsteigernde Elemente vorgesehen sein. An der dem Patienten 14 abgewandten Seite ist das Befestigungselement 54 zum Zusammenwirken mit den Befestigungselementen 52 angeordnet (nicht gezeigt). Dementsprechend können auch bei der Referenzierungsvorrichtung 60 die Markerelemente 34 individuell über eine Klettverbindung lösbar mit dem Schlauchkörper 62 verbunden werden.

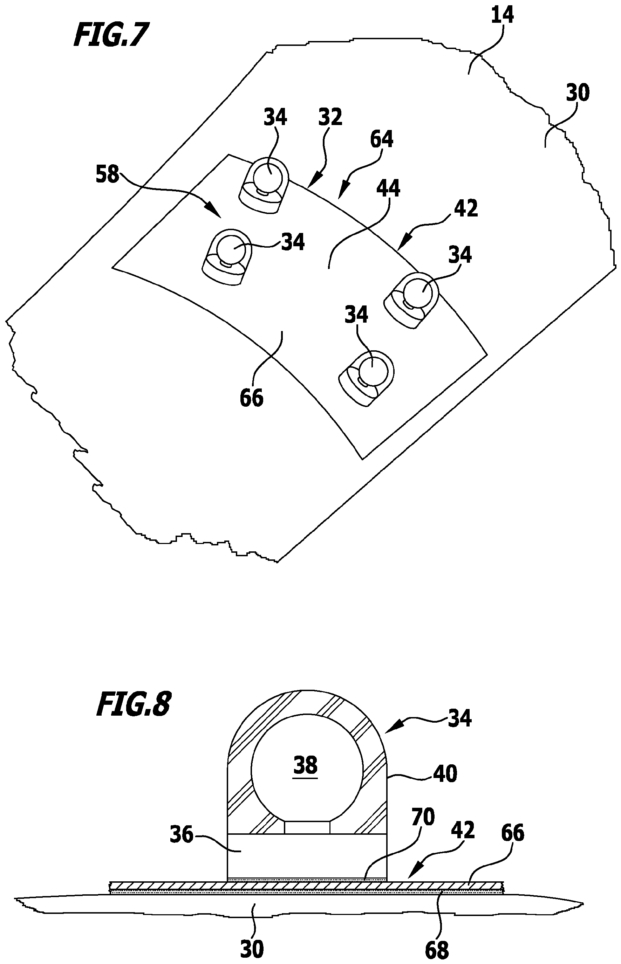

Die Figuren 7 und 8 zeigen eine mit dem Bezugszeichen 64 belegte Referenzierungsvorrichtung, ebenfalls umfassend vier Markerelemente 34. Die Befestigungseinrichtung 42 weist den Trägerkörper 44 auf. Der Trägerkörper 44 ist ausgestaltet als Aufkleber 66. Die Befestigungseinrichtung 42 umfasst eine Klebeschicht 68 am Aufkleber 66, über die dieser lösbar auf dem Patienten 14, insbesondere dem Oberschenkel 30, angebracht werden kann.

Die Befestigungseinrichtung 42 umfasst weiter ein Befestigungselement 70, ebenfalls ausgestaltet als Klebeschicht, an jedem Markerelement 34. Dies erlaubt es, die Markerelemente 34 klebend auf dem Aufkleber 66 zu befestigen und dabei individuell zu positionieren. Bevorzugt können die Markerelemente 34 vom Aufkleber 66 gelöst werden.

Alternativ zur Klebeschicht kann zum Befestigen der Markerelemente 34 am Aufkleber 66 eine Klettverbindung 56 zum Einsatz kommen.

Der Aufkleber 66 ist verformbar, wodurch er sich besonders gut an die Anatomie des Patienten 14 anpassen kann.

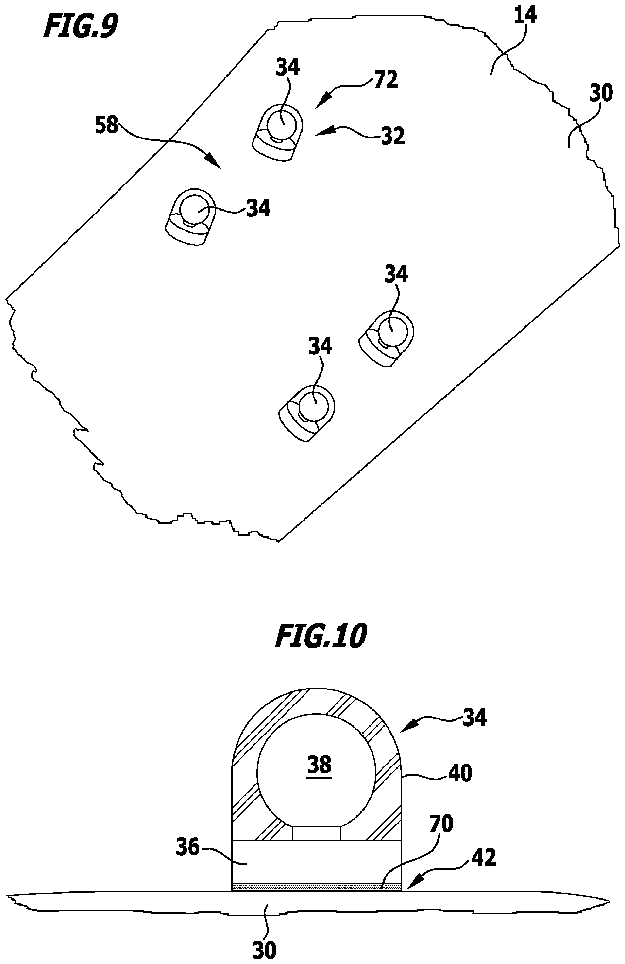

Die Figuren 9 und 10 zeigen eine mit dem Bezugszeichen 72 belegte, nicht beanspruchte Referenzierungsvorrichtung, die ebenfalls vier Markerelemente 34 umfasst. Bei der Referenzierungsvorrichtung 72 entfällt ein gemeinsamer Trägerkörper für die Markerelemente 34. Stattdessen können diese individuell am Patienten 14, insbesondere am Oberschenkel 30, befestigt werden. Zu diesem Zweck umfasst die Befestigungseinrichtung 42 an jedem Markerelement 34 das Befestigungselement 70, ausgestaltet als Klebeschicht.

Jedes Markerelement 34 kann individuell am Patienten 14 klebend fixiert werden. Aufgrund der Fähigkeit des Oberschenkels 30 zur Verformung sind auch die Markerelemente 34 relativ zueinander beweglich.

Es kann selbstverständlich vorgesehen sein, dass eine Mehrzahl von Markerelementen 34 über eine gemeinsame Klebeschicht 70 auf den Patienten 14 geklebt werden kann.

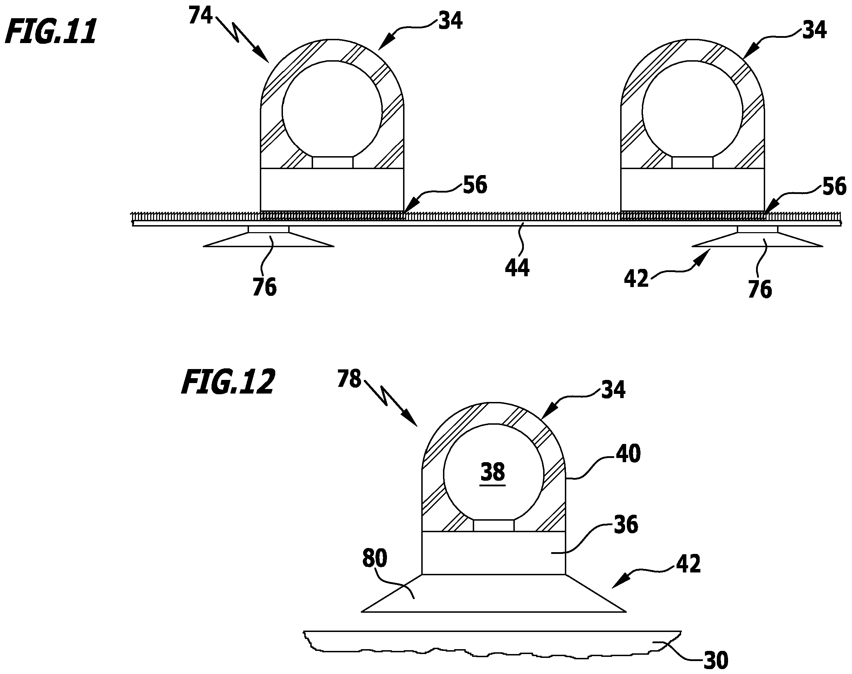

Figur 11 zeigt eine mit dem Bezugszeichen 74 belegte Referenzierungsvorrichtung, von der beispielhaft zwei Markerelemente 34 dargestellt sind. Die Markerelemente 34 sind über die Klettverbindung 56 mit dem Trägerkörper 44 (zum Beispiel der Bandage 46, dem Schlauchkörper 62 oder dem Aufkleber 66) verbunden, sie könnten jedoch auch über die Klebeschicht 70 auf diesem festgelegt sein.

Die Befestigungseinrichtung 42 umfasst bei der Referenzierungsvorrichtung 74 Befestigungselemente 76, die an der dem Patienten 14 zugewandten Seite am Trägerkörper 44 festgelegt sind. Die Befestigungselemente 76 sind ausgestaltet als Saugnäpfe. Über die Saugnäpfe 76 kann der Trägerkörper 44 handhabungsfreundlich lösbar am Patienten 14 festgelegt werden.

Von einer in Figur 12 teilweise dargestellten, nicht beanspruchten Referenzierungsvorrichtung 78 ist nur ein Markerelement 34 gezeigt. Die Referenzierungsvorrichtung 78 weist jedoch mehr Markerelemente 34 auf, beispielsweise vier Markerelemente 34.

Die Befestigungseinrichtung 42 umfasst ein Befestigungselement 80. Das Befestigungselement 80 ist ausgestaltet als Saugnapf, der an der dem Patienten zugewandten Seite des Grundelementes 36 angeordnet ist. Über den Saugnapf 80 kann das Markerelement 34 handhabungsfreundlich lösbar mit dem Patienten 14, insbesondere dem Oberschenkel 30, verbunden werden.

Als Saugnapf ausgestaltete Befestigungselemente können insbesondere auch bei Varianten der Referenzierungsvorrichtungen 64 und 74 zum Einsatz kommen.

Figur 13 zeigt in schematischer Draufsicht einen Ausschnitt eines Trägerkörpers 44 bei einer vorteilhaften Ausführungsform einer erfindungsgemäßen Referenzierungsvorrichtung. Beispielsweise handelt es sich bei dem Trägerkörper 44 um die Bandage 46 oder um den Schlauchkörper 62.

Der Trägerkörper 44 bildet einen Hohlraum 82 für ein Gas wie insbesondere Luft. Über ein Anschlusselement 84 am Trägerkörper 44 kann der Hohlraum 82 mit Unter- oder Überdruck beaufschlagt werden, wodurch die Formstabilität des Trägerkörpers 44 gesteigert werden kann. Am Anschlusselement 84 kann ein Ventilelement 86 vorhanden sein, um den Hohlraum 82 gasdicht zu verschließen.

Das Navigationssystem 10 kann eine Druckbeaufschlagungseinrichtung wie eine Saug- und/oder Druckpumpe umfassen. Über eine in der Zeichnung nicht dargestellte Saug- und/oder Druckleitung kann die Druckbeaufschlagungseinrichtung an das Anschlusselement 84 angeschlossen sein, wodurch der Trägerkörper 44 leer- oder aufgepumpt werden kann.

Beispielsweise kann vorgesehen sein, den Trägerkörper 44 ohne Druckbeaufschlagung am Patienten 14 zu fixieren. Anschließend kann der Trägerkörper 44 mit Unter- oder Überdruck beaufschlagt werden, um die Fixierung am Patienten 14 zu verbessern. Zugleich kann die Formstabilität des Trägerkörpers 44 gesteigert werden. Die Verformbarkeit des Trägerkörpers 44 wird dadurch verringert, wodurch die Beweglichkeit der Markerelemente 34 relativ zueinander ebenfalls verringert werden kann.

Die Referenzierungsvorrichtungen 60, 64, 72, 74 und/oder 78 können beim Navigationssystem 10 zum Einsatz kommen, anstelle der Referenzierungsvorrichtung 28 oder ergänzend zu dieser.

Bei allen Referenzierungsvorrichtungen 28, 60, 64, 72, 74 und 78 ist die von den Markerelementen 34 jeweils gebildete Markerelementanordnung 58 nicht-starr. Sie kann sich insbesondere aufgrund der Verformbarkeit der Bandage 46, des Schlauchkörpers 62, des Aufklebers 66 und der Verformbarkeit des Oberschenkels 30 verändern sowie, wie erwähnt, durch Repositionierung, Entfernen oder Hinzufügen eines oder mehrerer Markerelemente 34.

Alle Referenzierungsvorrichtungen 28, 60, 64, 72, 74 und 78 weisen den großen Vorteil auf, dass sie nicht-invasiv am Patienten 14 befestigbar sind, wodurch zusätzliches Trauma für den Patienten 14 insbesondere bei einer Hüft- oder Knieoperation vermieden werden kann.

Nachfolgend wird auf die Verfolgung der Referenzierungsvorrichtung 28 im Raum zur Bestimmung einer Änderung der Lage und Orientierung des Oberschenkels 30 im Raum eingegangen, wobei folgende Ausführungen im Falle einer Verwendung der Referenzierungsvorrichtung 60, 64, 72, 74 und/oder 78 ebenfalls gelten.

Trotz der Möglichkeit der Markerelemente 34, sich unter Änderung der Geometrie der Markerelementanordnung 58 bei der Referenzierungsvorrichtung 28 relativ zueinander zu bewegen, ist es für den Computer 18 möglich, auf Basis von Positionsdaten der Nachweisvorrichtung 24 die Lage- und Orientierungsänderung der Referenzierungsvorrichtung 28 und damit des Oberschenkels 30 zu bestimmen. Dies erfolgt insbesondere unter der Berücksichtigung der Annahme, dass die Relativbewegung der Markerelemente 34 zueinander möglich ist, im Allgemeinen jedoch geringer als eine Absolutbewegung der Markerelementanordnung 58 im Raum. Trotz nicht-invasiver Befestigung der Referenzierungsvorrichtung 28 kann dadurch dennoch zuverlässig eine hinreichende Bestimmung der Lage und Orientierung des Oberschenkels 30 ermittelt werden.

Bei dem Navigationssystem 10 ist insbesondere die Möglichkeit gegeben, dass der Computer 18 die Geometrie der Markerelementanordnung 58 gewissermaßen "automatisch" ermittelt, wenn die Referenzierungsvorrichtung 28 im Raum bewegt wird. Beispielsweise kann dem Benutzer auf dem Bildschirm 22 vor dem eigentlichen Eingriff ein Hinweis angezeigt werden, den Oberschenkel 30 mit daran angebrachter Bandage 46 zu bewegen. Der Computer 18 analysiert die Positionsdaten der Nachweisvorrichtung 24 und ist in der Lage, ohne vorherige Kenntnis des Vorhandenseins der Markerelemente 34, deren Anzahl und Relativanordnung, die Geometrie der Markerelementanordnung 58 zu bestimmen.

Die Geometrie der Markerelementanordnung 58 kann in einer Speichereinheit 88 des Computers gespeichert werden.

Bei der Bewegung des Oberschenkels 30 kann der Computer 18 ferner auf Basis der Positionsdaten die Eigenbeweglichkeiten der Markerelemente 34 ermitteln. Als Eigenbeweglichkeit eines Markerelementes 34 kann vorliegend insbesondere dessen Fähigkeit angesehen werden, sich innerhalb der Markerelementanordnung 58 relativ zu den anderen Markerelementen 34 zu bewegen. Die Bewegung eines Markerelementes erfolgt zum Beispiel durch eine Verformung der Bandage 46 in sich oder aufgrund einer Verformung des Oberschenkels 30 oder aufgrund der nicht-starren Befestigung des Markerelementes 34 an der Bandage 46.

Der Computer 18 kann zur Ermittlung der Eigenbeweglichkeit feststellen, in welchem Umfang sich das Markerelement 34 und/oder in welcher Raumrichtung sich dieses bewegen kann. Beispielsweise definiert der Computer 18 für jedes Markerelement 34 ein Beweglichkeitsellipsoid und nimmt für die Verfolgung der Referenzierungsvorrichtung 28 im Raum an, dass sich das Markerelement 34 normalerweise im Umfang dieses Beweglichkeitsellipsoides bewegt - bezogen auf eine ortsfeste Anordnung der Markerelementanordnung 58.

Auch die Eigenbeweglichkeiten der Markerelemente 34 können in der Speichereinheit 88 gespeichert werden. Sie werden bei einer Verfolgung der Referenzierungsvorrichtung 28 im Raum ebenso wie die Geometrie der Markerelementanordnung 58 zugrunde gelegt.

Der Computer 18 kann unter Berücksichtigung der Geometrie der Markerelementanordnung 58 und der Eigenbeweglichkeiten der Markerelemente 34 die Genauigkeit abschätzen, mit der eine zuverlässige Verfolgung der Referenzierungsvorrichtung 28 im Raum möglich ist. Stellt der Computer 18 fest, dass dies nicht der Fall zu sein scheint, kann er den Benutzer, beispielsweise über den Bildschirm 22, auf diesen Umstand hinweisen. Der Hinweis kann den Vorschlag umfassen, mindestens ein Markerelement 34 zu repositionieren oder mindestens ein Markerelement 34 hinzuzufügen. Im Falle einer solchen Repositionierung oder Ergänzung mindestens eines Markerelementes 34 kann das Navigationssystem die Markerelementanordnung 58 neu speichern und der weiteren Verfolgung der Referenzierungsvorrichtung 28 zugrunde legen.

Zur weiteren Unterstützung des Benutzers bereits bei der Anordnung und Fixierung der Referenzierungsvorrichtung 28 am Patienten 14 kann auf Erfahrungswissen zurückgegriffen werden, das im Navigationssystem 10 hinterlegt ist.

Beispielsweise sind in der Speichereinheit 88 Muster (sogenannte "Templates") gespeichert für Markerelementanordnungen hinsichtlich der Anzahl und/oder der Relativanordnung der Markerelemente 34 und/oder deren Positionierung am Trägerkörper 44 zur Bildung einer vorgebbaren Geometrie der Markerelementanordnung 58. Ferner kann in einem jeweiligen Muster Information betreffend die Art oder Anordnung der Referenzierungsvorrichtung 28 am Patienten 14 gespeichert sein oder Information betreffend die Vorteile einer jeweiligen Referenzierungsvorrichtung 28, 60, 64, 72, 74 oder 78.

Basierend auf in einem derartigen Muster gespeicherten Vorwissen kann der Computer 18 den Operateur über den Bildschirm 22 anleiten und dabei unterstützen, die Referenzierungsvorrichtung 28 (oder eine der anderen Referenzierungsvorrichtungen) sowie insbesondere deren Markerelemente 34 so am Patienten 14 zu befestigen, dass die Geometrie der Markerelementanordnung 58 vom Navigationssystem 10 voraussichtlich zuverlässig erkannt werden kann.

Bei der Verfolgung der Referenzierungsvorrichtung 28 im Raum kann der Computer 18 bei der Analyse der Positionsdaten Bewegungen der Markerelemente 34 innerhalb der Markerelementanordnung 58 dadurch kompensieren, dass die Eigenbeweglichkeiten berücksichtigt werden. Insbesondere ist die Möglichkeit gegeben, dass die Positionsdaten abhängig von der Größe der jeweiligen Eigenbeweglichkeit unterschiedlich gewichtet werden. Beispielsweise werden Positionsdaten eines Markerelementes 34, das eine verhältnismäßig geringe Eigenbeweglichkeit aufweist, als vertrauenswürdiger angesehen als Positionsdaten eines Markerelementes 34, dessen Eigenbeweglichkeit verhältnismäßig groß ist. Dies erlaubt es dem Computer 18, ein Maß für die Zuverlässigkeit und für die Bestimmbarkeit bei der Verfolgung der Referenzierungsvorrichtung 28 im Raum zu erstellen.

Bei der Verfolgung der Referenzierungsvorrichtung 28 im Raum, um deren Lage- und/oder Orientierungsänderung zu bestimmen, werden von der Nachweisvorrichtung 24 in aufeinanderfolgenden zeitlichen Abständen (beispielsweise mit einer Frequenz von 25 Hz) Datensätze erstellt. Diese werden hinsichtlich der Positionsdaten der Markerelemente 34 vom Computer 18 untersucht. Der Computer 18 kann zum Beispiel feststellen, ob die Bewegung eines Markerelementes 34 innerhalb der Markerelementanordnung 58 eine Schwellenwertbewegung überschreitet. Als Schwellenwertbedingung kann zum Beispiel die zuvor ermittelte Eigenbeweglichkeit des Markerelementes 34 herangezogen werden.

Ist dies der Fall, kann der Computer 18 bestimmen, dass Positionsdaten des entsprechenden Markerelementes 34 eine geringere Gewichtung erhalten, um die Referenzierungsvorrichtung 28 im Raum zu identifizieren. Gegebenenfalls können Positionsdaten des Markerelementes 34 für eine laufende Bestimmung sogar gänzlich ignoriert werden. Dies ist insbesondere dann möglich, wenn anhand der Positionsdaten der übrigen Markerelemente 34 eine eindeutige Bestimmung der Lage und der Orientierung der Markerelementanordnung 58 und damit der Referenzierungsvorrichtung 28 im Raum möglich ist.

In entsprechender Weise können die Positionsdaten von Markerelementen, deren Bewegung innerhalb der Markerelementanordnung 58 eine Schwellenwertbewegung unterschreitet, als besonders vertrauenswürdig herangezogen werden. Ein derartiges Markerelement 34 kann beispielsweise als Referenzmarkerelement herangezogen werden, anhand von dessen Positionsdaten eine Absolutbewegung der Markerelementanordnung 58 im Raum abgeleitet werden kann.

Der Computer 18 kann die Eigenbeweglichkeiten der Markerelemente 34 während der Verfolgung der Referenzierungsvorrichtung 28 überwachen und erforderlichenfalls aktualisieren, so dass diese weiteren Berechnungen zugrunde gelegt werden können.

In entsprechender Weise kann auch während der Verfolgung der Referenzierungsvorrichtung am Bildschirm 22 ein Hinweis an den Benutzer ausgegeben werden, mindestens ein Markerelement 34 zu repositionieren oder mindestens ein Markerelement 34 hinzuzufügen, mit dem Ziel, eine möglichst genaue Referenz zu erzielen.

## Patentansprüche

1. Chirurgische Referenzierungsvorrichtung, die von einem chirurgischen Navigationssystem verfolgt werden kann, umfassend eine Markiereinrichtung (32) mit mindestens zwei oder mehr chirurgischen Markerelementen (34), die zum Reflektieren von Strahlung ausgebildet sind, wobei die Markerelemente (34) eine Markerelementanordnung (58) ausbilden, die eine Referenz am Körper eines Patienten (14) definiert, sowie umfassend eine Befestigungseinrichtung (42) zum Festlegen der Markiereinrichtung (32) am Körper des Patienten (14), **dadurch gekennzeichnet, dass** die Markerelementanordnung (58) nicht-starr ausgestaltet ist und zwei oder mehr Markerelemente (34) positionsveränderlich zueinander sind, wobei die Befestigungseinrichtung (42) einen einer Mehrzahl der Markerelemente (34) zugeordneten formveränderlichen, an die Anatomie des Patienten (14) anpassbaren Trägerkörper (44) aufweist oder ausbildet, an dem die Markerelemente (34) lösbar festlegbar sind.

2. Referenzierungsvorrichtung nach Anspruch 1, **dadurch gekennzeichnet, dass** die Markerelemente (34) gruppenweise positionsveränderlich zueinander sind, wobei eine Gruppe von Markerelementen (34) mindestens zwei Markerelemente (34) umfasst und zwei oder mehr Gruppen von Markerelementen (34) vorhanden sind.

3. Referenzierungsvorrichtung nach einem der voranstehenden Ansprüche, **dadurch gekennzeichnet, dass** die Befestigungseinrichtung (42) so ausgebildet ist, dass die Markiereinrichtung (32) nicht-invasiv am Körper des Patienten (14) festlegbar ist.

4. Referenzierungsvorrichtung nach einem der voranstehenden Ansprüche, **dadurch gekennzeichnet, dass** die Befestigungseinrichtung (42) an einem oder an mehreren Markerelementen (34) ein Befestigungselement (52, 70) umfasst.

5. Referenzierungsvorrichtung nach Anspruch 4, **dadurch gekennzeichnet,**
- **dass** mindestens ein Befestigungselement (70) eine Klebeschicht umfasst; und/oder
- **dass** mindestens ein Befestigungselement (70) ein Flausch- oder Hakenband ist zum Zusammenwirken mit einem Haken- bzw. Flauschband am Trägerkörper (44); und/oder
- **dass** die Befestigungseinrichtung (42) an einem oder mehreren Markerelementen (34) ein Befestigungselement umfasst, das ein Saugnapf (76) ist oder einen solchen umfasst.

6. Referenzierungsvorrichtung nach einem der voranstehenden Ansprüche, **dadurch gekennzeichnet, dass** die Markerelemente (34) getrennt voneinander am Trägerkörper (44) positionierbar und/oder individuell am Trägerkörper (44) festlegbar sind.

7. Referenzierungsvorrichtung nach einem der voranstehenden Ansprüche, **dadurch gekennzeichnet, dass** der Trägerkörper (44) Folgendes ist oder umfasst:
- ein(en) Textilverbund; und/oder
- eine Bandage (46); und/oder
- ein(en) Schlauch- oder Strumpfkörper (62).

8. Referenzierungsvorrichtung nach einem der voranstehenden Ansprüche, **dadurch gekennzeichnet, dass** der Trägerkörper (44) auf dem Patientenkörper aufklebbar ist und/oder mittels mindestens eines am Trägerkörper (44) angeordneten Saugnapfes (76) am Patientenkörper festlegbar ist.

9. Referenzierungsvorrichtung nach einem der voranstehenden Ansprüche, **dadurch gekennzeichnet, dass** der Trägerkörper (44) eine folienförmige Ausgestaltung aufweist.

10. Chirurgisches Navigationssystem zum Verfolgen einer chirurgischen Referenzierungsvorrichtung (28; 60; 64; 72; 74; 78) im Raum, wobei das Navigationssystem (10) eine Referenzierungsvorrichtung (28; 60; 64; 72; 74; 78) mit einer Markiereinrichtung (32) aufweisend mindestens zwei oder mehr Markerelemente (34) umfasst, eine Nachweisvorrichtung (24) zum Detektieren von von den Markerelementen (34) reflektierter Strahlung und Bereitstellen diesbezüglicher Positionsdaten sowie eine Datenverarbeitungsvorrichtung (16) zur Verarbeitung der Positionsdaten und darauf beruhender Ermittlung der Lage und der Orientierung der Referenzierungsvorrichtung (28; 60; 64; 72; 74; 78) im Raum, wobei die Referenzierungsvorrichtung (28; 60; 64; 72; 74; 78) eine Referenzierungsvorrichtung (28; 60; 64; 72; 74; 78) nach einem der voranstehenden Ansprüche ist.

11. Navigationssystem nach Anspruch 10, **dadurch gekennzeichnet, dass** die Datenverarbeitungsvorrichtung (16) so ausgebildet und programmiert ist, dass sie ermittelt, welche Eigenbeweglichkeit die Markerelemente (34) innerhalb der Markerelementanordnung (58) aufweisen und dass die Datenverarbeitungsvorrichtung (16) in einer Speichereinheit die jeweilige Eigenbeweglichkeit speichert oder eine bereits gespeicherte Eigenbeweglichkeit aktualisiert und bei einer nachfolgenden Ermittlung der Änderung der Lage und der Orientierung der Referenzierungsvorrichtung (28; 60; 64; 72; 74; 78) im Raum zugrunde legt.

12. Navigationssystem nach Anspruch 11, **dadurch gekennzeichnet, dass** die Datenverarbeitungsvorrichtung (16) so ausgebildet und programmiert ist,
- dass sie Positionsdaten von Markerelementen (34) abhängig von der Größe der jeweiligen Eigenbeweglichkeit bei einer Ermittlung der Änderung der Lage und Orientierung der Referenzierungsvorrichtung (28; 60; 64; 72; 74; 78) im Raum unterschiedlich gewichtet, wobei Markerelemente (34) mit geringer Eigenbeweglichkeit eine höhere Gewichtung erhalten als Markerelemente (34) größerer Eigenbeweglichkeit; und/oder
- dass sie unter Berücksichtigung der Eigenbeweglichkeiten der Markerelemente (34) die Genauigkeit einschätzt, eine Änderung der Lage und der Orientierung der Referenzierungsvorrichtung (28; 60; 64; 72; 74; 78) im Raum ermitteln zu können, und dass die Datenverarbeitungsvorrichtung (16) bei nicht hinreichender Genauigkeit einen Hinweis an einer Hinweiseinheit (20) des Navigationssystems (10) vorschlagend die Änderung der Position mindestens eines Markerelementes (34) oder die Hinzufügung mindestens eines weiteren Markerelementes (34) zur Markerelementanordnung (58) ausgibt.

13. Navigationssystem nach einem der Ansprüche 10 bis 12, **dadurch gekennzeichnet, dass** die Datenverarbeitungsvorrichtung so ausgebildet und programmiert ist, dass sie bei Kenntnis der Lage der Referenzierungsvorrichtung (28; 60; 64; 72; 74; 78) relativ zu einer körpereigenen Struktur oder durch diese definierten Achse, Fläche oder Ebene des Patienten (14) die Genauigkeit einschätzt, eine Änderung der Lage der körpereigenen Struktur, Achse, Fläche oder Ebene im Raum ermitteln zu können, und dass die Datenverarbeitungsvorrichtung (16) bei nicht hinreichender Genauigkeit einen Hinweis an einer Hinweiseinheit (20) des Navigationssystems (10) vorschlagend die Änderung der Position mindestens eines Markerelementes oder die Hinzufügung mindestens eines weiteren Markerelementes (34) zur Markerelementanordnung (58) ausgibt.

14. Navigationssystem nach einem der Ansprüche 10 bis 13, **dadurch gekennzeichnet, dass** die Datenverarbeitungsvorrichtung (16) so ausgebildet und programmiert ist, dass sie an einer Hinweiseinheit (20) des Navigationssystems (10) einen Hinweis für den Benutzer ausgibt betreffend die Anzahl und/oder die Position der einzusetzenden Markerelemente (34) bei deren Anordnung am Körper des Patienten (14), insbesondere den Abstand der Markerelemente (34) voneinander und/oder deren Anordnung relativ zueinander und/oder einen Hinweis betreffend die Art und Weise der Befestigung der Markerelemente (34) am Körper des Patienten (14) über die Befestigungseinrichtung (42).

## Claims

1. Surgical referencing apparatus, which can be tracked by a surgical navigation system, comprising a marking device (32) having at least two or more surgical marker elements (34) which are configured for reflecting radiation, wherein the marker elements (34) form a marker element arrangement (58), which defines a reference on the body of a patient (14), and comprising a fixing device (42) for fixing the marking device (32) in place on the body of the patient (14), **characterized in that** the marker element arrangement (58) is of non-rigid configuration, and two or more marker elements (34) are variable in position relative to one another, wherein the fixing device (42) comprises or forms a carrier body (44) that is associated with a plurality of the marker elements (34) and is variable in shape and adjustable to the anatomy of the patient, on which carrier body (44) the marker elements (34) are removably fixable in place.

2. Referencing apparatus in accordance with claim 1, **characterized in that** the marker elements (34) are variable in position relative to each other in groups, wherein a group of marker elements (34) comprises at least two marker elements (34) and wherein two or more groups of marker elements (34) are provided.

3. Referencing apparatus in accordance with any one of the preceding claims, **characterized in that** the fixing device (42) is configured such that the marking device (32) is fixable in place on the body of the patient (14) in a non-invasive manner.

4. Referencing apparatus in accordance with any one of the preceding claims, **characterized in that** the fixing device (42) comprises a fixing element (52, 70) on one or more marker elements (34).

5. Referencing apparatus in accordance with claim 4, **characterized in that**
- at least one fixing element (70) comprises an adhesive layer; and/or
- at least one fixing element (70) is a loop tape or a hook tape for cooperating with a hook tape and a loop tape respectively on a carrier body (44); and/or
- the fixing device (42) comprises a fixing element on one or more marker elements (34), which fixing element is or comprises a suction cup (76).

6. Referencing apparatus in accordance with any one of the preceding claims, **characterized in that** the marker elements (34) are positionable on the carrier body in a manner separate from one another and/or individually fixable on a carrier body (44).

7. Referencing apparatus in accordance with any one of the preceding claims, **characterized in that** the carrier body (44) is or comprises the followi ng:
- a textile composite; and/or
- a bandage (46); and/or
- a tubular or stocking body (62).

8. Referencing apparatus in accordance with any one of the preceding claims, **characterized in that** the carrier body (44) is adhesively connectable to the patient's body and/or is fixable on the patient's body by means of at least one suction cup (76) arranged on the carrier body (44).

9. Referencing apparatus in accordance with any one of the preceding claims, **characterized in that** the carrier body (44) is of sheet-like configuration.

10. Surgical navigation system for tracking a surgical referencing apparatus (28; 60; 64; 72; 74; 78) in space, wherein the navigation system (10) comprises a referencing apparatus (28; 60; 64; 72; 74; 78) comprising a marking device (32) having at least two or more marker elements (34), a detection apparatus (24) for detecting radiation reflected by the marker elements (34) and for providing positional data related thereto, and a data processing apparatus (16) for processing the positional data and for determining, on the basis thereof, the position and orientation of the referencing apparatus (28; 60; 64; 72; 74; 78) in space, wherein the referencing apparatus (28; 60; 64; 72; 74; 78) is a referencing apparatus (28; 60; 64; 72; 74; 78) in accordance with any one of the preceding claims.

11. Navigation system in accordance with claim 10, **characterized in that** the data processing apparatus (16) is configured and programmed such that it determines what own mobility the marker elements (34) have within the marker element arrangement (58) and such that the data processing apparatus (16) stores the respective own mobility in a storage unit or updates an already stored own mobility and uses this as a basis for a subsequent determination of the change in the position and orientation of the referencing apparatus (28; 60; 64; 72; 74; 78) in space.

12. Navigation system in accordance with claim 11, **characterized in that** the data processing apparatus (16) is configured and programmed such
- that in a determination of the change in the position and orientation of the referencing apparatus (28; 60; 64; 72; 74; 78) in space, it gives different weighting to positional data of marker elements (34) depending on the magnitude of the respective own mobility thereof, wherein marker elements (34) that have low own mobility are given a higher weighting than marker elements (34) having a larger own mobility; and/or
- that taking into account the own mobilities of the marker elements (34), it estimates the accuracy with which it is able to determine a change in the position and orientation of the referencing apparatus (28; 60; 64; 72; 74; 78) in space, and such that, if there is insufficient accuracy, the data processing apparatus (16) outputs on an indication unit (20) of the navigation system (10) an indication proposing to change the position of at least one marker element (34) or to add at least one further marker element (34) to the marker element arrangement (58).

13. Navigation system in accordance with any one of claims 10 to 12, **characterized in that** the data processing apparatus is configured and programmed such that, when the position of the referencing apparatus (28; 60; 64; 72; 74; 78) relative to a body's own structure or an axis, surface or plane of the patient (14) defined thereby is known, it estimates the accuracy with which it is able to determine a change in the position of the body's own structure, axis, surface or plane in space, and such that, if there is insufficient accuracy, the data processing apparatus (16) outputs on an indication unit (20) of the navigation system (10) an indication proposing to change the position of at least one marker element or to add at least one further marker element (34) to the marker element arrangement (58).

14. Navigation system in accordance with any one of claims 10 to 13, **characterized in that** the data processing apparatus (16) is configured and programmed such that it outputs on an indication unit (20) of the navigation system (10) an indication to the user regarding the number and/or the position of the marker elements (34) to be used in the arrangement thereof on the body of the patient (14), in particular regarding the distance of the marker elements (34) from one another and/or their arrangement relative to one another, and/or an indication regarding how to fix the marker elements (34) to the body of the patient (14) via the fixing device (42).

## Revendications

1. Dispositif de référencement chirurgical qui peut être suivi par un système de navigation chirurgical, comprenant un organe de marquage (32) avec au moins deux ou plusieurs éléments de marquage chirurgicaux (34) qui sont conçus pour réfléchir le rayonnement, où les éléments de marquage (34) forment un agencement d'éléments de marquage (58) qui définit une référence sur le corps d'un patient (14), et comprenant un organe de fixation (42) pour fixer l'organe de marquage (32) sur le corps du patient (14), **caractérisé en ce que** l'agencement d'éléments de marquage (58) est conçu de manière non rigide et deux ou plusieurs éléments de marquage (34) sont modifiables en position les uns par rapport aux autres, où l'organe de fixation (42) présente ou forme un corps de support (44) adaptable à l'anatomie du patient (14), de forme modifiable, associé à une pluralité des éléments de marquage (34), auquel les éléments de marquage (34) peuvent être fixés de manière amovible.

2. Dispositif de référencement selon la revendication 1, **caractérisé en ce que** les éléments de marquage (34) sont modifiables en position les uns par rapport aux autres en groupes, où un groupe d'éléments de marquage (34) comprend au moins deux éléments de marquage (34) et deux ou plusieurs groupes d'éléments de marquage (34) sont présents.

3. Dispositif de référencement selon l'une des revendications précédentes, **caractérisé en ce que** l'organe de fixation (42) est conçu de telle manière que l'organe de marquage (32) peut être fixé de manière non invasive sur le corps du patient (14).

4. Dispositif de référencement selon l'une des revendications précédentes, **caractérisé en ce que** l'organe de fixation (42) comprend sur un ou plusieurs éléments de marquage (34) un élément de fixation (52, 70).

5. Dispositif de référencement selon la revendication 4, **caractérisé**
- **en ce qu'**au moins un élément de fixation (70) comprend une couche adhésive ; et/ou
- **en ce qu'**au moins un élément de fixation (70) est une bande à boucles ou à crochets pour l'action conjointe avec une bande à crochets ou à boucles sur le corps de support (44) ; et/ou
- **en ce que** l'organe de fixation (42) comprend sur un ou plusieurs éléments de marquage (34) un élément de fixation qui est ou comprend une ventouse (76).

6. Dispositif de référencement selon l'une des revendications précédentes, **caractérisé en ce que** les éléments de marquage (34) peuvent être positionnés séparément les uns des autres sur le corps de support (44) et/ou peuvent être fixés individuellement sur le corps de support (44).

7. Dispositif de référencement selon l'une des revendications précédentes, **caractérisé en ce que** le corps de support (44) est ou comprend ce qui suit :
- un composite textile ; et/ou
- un bandage (46) ; et/ou
- un corps de type tuyau ou bas (62).

8. Dispositif de référencement selon l'une des revendications précédentes, **caractérisé en ce que** le corps de support (44) peut être collé sur le corps du patient et/ou peut être fixé sur le corps du patient au moyen d'au moins une ventouse (76) disposée sur le corps de support (44).

9. Dispositif de référencement selon l'une des revendications précédentes, **caractérisé en ce que** le corps de support (44) présente une configuration en forme de feuille.

10. Système de navigation chirurgical pour suivre un dispositif de référencement chirurgical (28 ; 60 ; 64 ; 72 ; 74 ; 78) dans l'espace, où le système de navigation (10) comprend un dispositif de référencement (28 ; 60 ; 64 ; 72 ; 74 ; 78) avec un organe de marquage (32) présentant au moins deux ou plusieurs éléments de marquage (34), un dispositif de détection (24) pour détecter le rayonnement réfléchi par les éléments de marquage (34) et fournir des données de position correspondantes ainsi qu'un dispositif de traitement de données (16) pour traiter les données de position et déterminer sur cette base la position et l'orientation du dispositif de référencement (28 ; 60 ; 64 ; 72 ; 74 ; 78) dans l'espace, où le dispositif de référencement (28 ; 60 ; 64 ; 72 ; 74 ; 78) est un dispositif de référencement (28 ; 60 ; 64 ; 72 ; 74 ; 78) selon l'une des revendications précédentes.

11. Système de navigation selon la revendication 10, **caractérisé en ce que** le dispositif de traitement de données (16) est conçu et programmé de telle manière qu'il détermine quelle mobilité intrinsèque les éléments de marquage (34) présentent au sein de l'agencement d'éléments de marquage (58) et que le dispositif de traitement de données (16) mémorise la mobilité intrinsèque respective dans une unité de mémoire ou actualise une mobilité intrinsèque déjà mémorisée et l'utilise comme base lors d'une détermination ultérieure du changement de position et d'orientation du dispositif de référencement (28 ; 60 ; 64 ; 72 ; 74 ; 78) dans l'espace.

12. Système de navigation selon la revendication 11, **caractérisé en ce que** le dispositif de traitement de données (16) est conçu et programmé,
- de telle manière qu'il pondère différemment des données de position d'éléments de marquage (34) en fonction de l'ampleur de la mobilité intrinsèque respective lors d'une détermination du changement de position et d'orientation du dispositif de référencement (28 ; 60 ; 64 ; 72 ; 74 ; 78) dans l'espace, où les éléments de marquage (34) ayant une faible mobilité intrinsèque acquièrent une pondération plus élevée que les éléments de marquage (34) de plus grande mobilité intrinsèque ; et/ou
- de telle manière qu'en tenant compte des mobilités intrinsèques des éléments de marquage (34), il évalue la précision de pouvoir déterminer un changement de position et d'orientation du dispositif de référencement (28 ; 60 ; 64 ; 72 ; 74 ; 78) dans l'espace, et que le dispositif de traitement de données (16), si la précision est insuffisante, délivre une information à une unité d'information (20) du système de navigation (10) proposant le changement de position d'au moins un élément de marquage (34) ou l'ajout d'au moins un autre élément de marquage (34) à l'agencement d'éléments de marquage (58).

13. Système de navigation selon l'une des revendications 10 à 12, **caractérisé en ce que** le dispositif de traitement de données est conçu et programmé de telle manière que, lorsque la position du dispositif de référencement (28 ; 60 ; 64 ; 72 ; 74 ; 78) par rapport à une structure intrinsèque du corps ou à un axe, une surface ou un plan du patient (14) défini par celle-ci est connue, il évalue la précision de pouvoir déterminer un changement de la position de la structure intrinsèque du corps, de l'axe, de la surface ou du plan dans l'espace, et le dispositif de traitement de données (16), si la précision n'est pas suffisante, délivre une information à une unité d'information (20) du système de navigation (10) proposant le changement de position d'au moins un élément de marquage ou l'ajout d'au moins un autre élément de marquage (34) à l'agencement d'éléments de marquage (58).

14. Système de navigation selon l'une des revendications 10 à 13, **caractérisé en ce que** le dispositif de traitement de données (16) est conçu et programmé de telle manière qu'il délivre à une unité d'information (20) du système de navigation (10) une information pour l'utilisateur concernant le nombre et/ou la position des éléments de marquage (34) à utiliser lors de leur agencement sur le corps du patient (14), en particulier la distance des éléments de marquage (34) entre eux et/ou leur agencement les uns par rapport aux autres et/ou une information concernant le type et le mode de fixation des éléments de marquage (34) sur le corps du patient (14) par le biais de l'organe de fixation (42).
